# EUROPEAN PATENT APPLICATION

(11) **EP 2 446 928 A1**
(43) Date of publication of application: **02.05.2012**
(21) Application number: 10815423.8
(22) Date of filing: 09.09.2010
(51) Int. Cl.: A61N 5/06

(54) **METHOD FOR KILLING TUMOR CELLS SELECTIVELY AND APPARATUS FOR THE METHOD**

(30) Priority: 09.09.2009 JP 2009208009
(71) Applicant: Tokai University Educational System, Tokyo 151-0063 (JP); Comet Corporation, Tokyo 102-0071 (JP)
(72) Inventor: ITOH, Johbu, Isehara-shi Kanagawa 259-1193 (JP)
(74) Representative: Hall, Matthew Benjamin
(86) International application number: PCT/JP2010/065534
(87) International publication number: WO 2011/030828

(57) **Abstract**

It is an object of the present invention to provide a method and an apparatus capable of selectively damaging and killing tumor cells. In one aspect of the present invention, there is provided a method for selectively damaging and killing tumor cells comprising a step of irradiating tumor cells with a pulse light having continuous emission spectra ranging at least from 230 to 270 nm (UV pulse flash), outside a living body of a human or a living body of a non-human animal or in a living body of a non-human animal. The UV pulse flash, preferably, has an accumulated irradiation amount per unit area that is achieved at a distance of 8 cm from a light source having an integrated output of, for example, 90, 180-7100 or 14200 J. In other words, the UV pulse flash, preferably, has an accumulated irradiation amount per unit area of, for example, 6, 12-480 or 960 J/cm² in terms of an energy originated from a wavelength of UVC. The step of irradiating with the UV pulse flash is preferably carried out, for example, within 1 minute. The UV pulse flash is preferably emitted from a xenon flash lamp. As another aspect of the present invention, there is provided an apparatus for treating tumor tissues comprising a light source of the UV pulse flash.

## Description

### Technical Field

The present invention relates to a method and an apparatus for selectively damaging and killing tumor cells using a specific UV pulse flash.

### Background Art

Conventional known methods for disrupting cancer cells include radiation methods using radiation, gamma ray or baryon beam. These methods, however, have problems that it is difficult to perform pinpoint treatment at tumor cancer tissues present deep in a living body, and these methods do damage to normal tissues in addition to tumor cells.

It is well known that ultraviolet ray has a sterilizing effect, and a low-pressure mercury lamp (UV lamp) has been long used as a sterilizing lamp. In recent years, a "light pulse sterilization" using a xenon flash lamp has come to be used (for example, see Patent Document 1). The xenon flash lamp can emit a light having a wavelength spectra ranging from 200 to 300 nm, at which range the sterilizing effect is supposedly strong, instantaneously at a microsecond order interval (several times to some dozen times per one second). Per one emission, the xenon flash lamp provides an energy in an amount tens of thousands times as high as that provided by the UV lamp (which provides about 65 W). The light pulse sterilization using the xenon flash lamp is a method achieving high sterilizing power against general bacteria, fungus, spore forming bacteria and the like for an extremely short period of time (not more than one second to about several seconds). Practical use of this method has started in the field of food and the like.

However, the applicability of the pulse light employing the xenon flash lamp or the like to the selective damaging and killing of the tumor cells has not been reported.

### Citation List

### Patent Document

Patent Document 1: JP-A-2000-107262

### Summary of the Invention

### Problem to be Solved by the Invention

It is an object of the present invention to provide a method and an apparatus capable of selectively damaging and killing tumor cells.

### Means for Solving the Problem

The present inventors found that the irradiation of tumor cells and non-tumor cells with a pulse light to be emitted from a xenon flash lamp or the like makes it possible to selectively damage and kill the tumor cells alone causing cell death to the tumor cells, but to allow the non-tumor cells to be viable. The present invention has been completed based on this finding.

That is, in one aspect of the present invention, there is provided a method for selectively damaging and killing tumor cells comprising a step of irradiating tumor cells with a pulse light having continuous emission spectra ranging at least from 230 to 270 nm (hereinafter, also referred to as a "UV pulse flash"), outside a living body of a human or a living body of a non-human animal or in a living body of a non-human animal. Hereinafter, such a method for selectively damaging and killing tumor cells is also referred to simply as the "method of the present invention".

The UV pulse flash, preferably, has an accumulated irradiation amount per unit area that is achieved at a distance of 8 cm from a light source having an integrated output of, for example, 90 to 7100 J, 90 to 14200 J, or 180 to 14200 J, according to tumor cells to be targeted. In other words, the UV pulse flash, preferably, has an accumulated irradiation amount per unit area of, for example, 6 to 480 J/cm², 6 to 960 J/cm², or 12 to 960 J/cm², in terms of an energy originated from a wavelength of UVC. The step of irradiating with the UV pulse flash is preferably carried out, for example, within 1 minute. The UV pulse flash is preferably emitted from a xenon flash lamp.

As another aspect of the present invention, there is provided an apparatus for medical treatment which is capable of performing the above method for selectively damaging and killing tumor cells, i.e., an apparatus for treating tumor tissues comprising a light source of a pulse light having continuous emission spectra ranging at least from 230 to 270 nm (UV pulse flash). Hereinafter, such an apparatus is also referred to as the "apparatus of the present invention".

As the apparatus for treating tumor tissues, there can be mentioned, for example, an endoscope, a laser microscope, or a body surface tumor tissue irradiating apparatus. As a light source to be possessed by such an apparatus, a xenon flash lamp is preferable.

### Effect of the Invention

With the method and the apparatus of the present invention, it is possible to eliminate tumor cells alone without causing cell death to non-tumor cells, with ease for a short period of time. By the present invention, the application to pinpoint treatment of tumor cancer tissues present in a living body is expected.

### Brief Description of Drawings

[Fig. 1] (a) Spectra in a UV region (200 to 400 nm) and (b) Spectra in a UV region, a visible region and an infrared region (200 to 950 nm) of a UV pulse flash to be emitted from a xenon flash lamp (BHX-200, COMET Corp.) at a light source, after transmitting through a normal glass (thickness: 0.17 mm) (not a quartz glass), and after transmitting through a plastic dish (FluoroDish FD-35, thickness: 1.25 mm)
[Fig. 2] Images of MCF-7 (tumor cell) observed by SEM in Example 1
   [a] The frequency of irradiation: 0 (control) Cell membrane has a smooth surface.
   [b] The frequency of irradiation: 56 times (1 sec, 179.2 J) Cytoplasm atrophy is recognized.
   [c] The frequency of irradiation: 280 times (5 sec, 896 J) Cytoplasm atrophy and membrane degeneration are recognize.
   [d] The frequency of irradiation: 560 times (10 sec, 1792 J) Individual cell morphology is not recognized. Membrane degeneration causes the cell surface to have projections.
   [e] The frequency of irradiation: 1120 times (20 sec, 3584 J) With the progression of cytoclasis, morphology is not partially formed.
   [f] The frequency of irradiation: 2240 times (40 sec, 7168 J) With a nucleus structure alone being recognized, cytoclasis is progressed.
   [g] The frequency of irradiation: 2240 times (40 sec, 7168 J), UV-C cut Removing UV-C, cell membrane having a smooth surface is observed, similarly to the control.

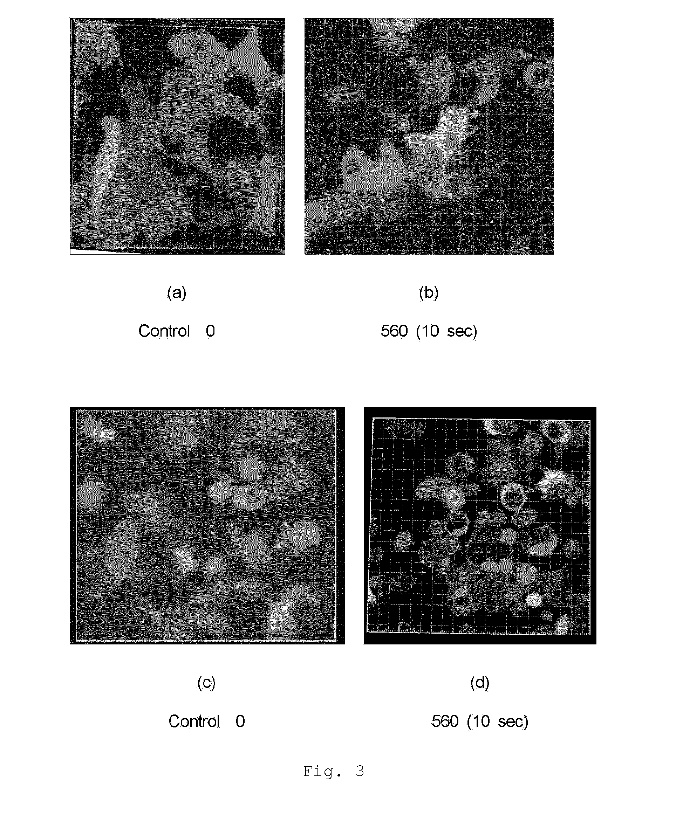

[Fig. 3] Images of Cos7 (non-tumor cell) and MCF-7 (tumor cell) observed by a laser microscope (LSM510-META) in the measurement of cell viability in Example 1 The life and death of cells is determined based on PI (propidium iodide): A living cell, because of having a solid membrane structure, cannot allow PI to infiltrate into the cell, consequently not dyeing red. A dead cell, having its membrane structure broken, allows PI to infiltrate into the cell and is reacted with DNA, consequently dyeing red.
   [a] Cos7/24 hours after no-irradiation (control) A large and flat cytoplasm is observed. No dead cell is recognized.
   [b] Cos7/24 hours after irradiation (the frequency of irradiation: 560 times) (10 sec, 1792 J) Cell atrophy is recognized, but no dead cell is recognized.
   [c] MCF-7/24 hours after no-irradiation (control) A round cell morphology specific to MCF-7 is observed.
   [d] MCF-7/24 hours after irradiation (the frequency of irradiation: 560 times) (10 sec, 1792 J) Cytoplasm atrophy is recognized, and all cells are eliminated.

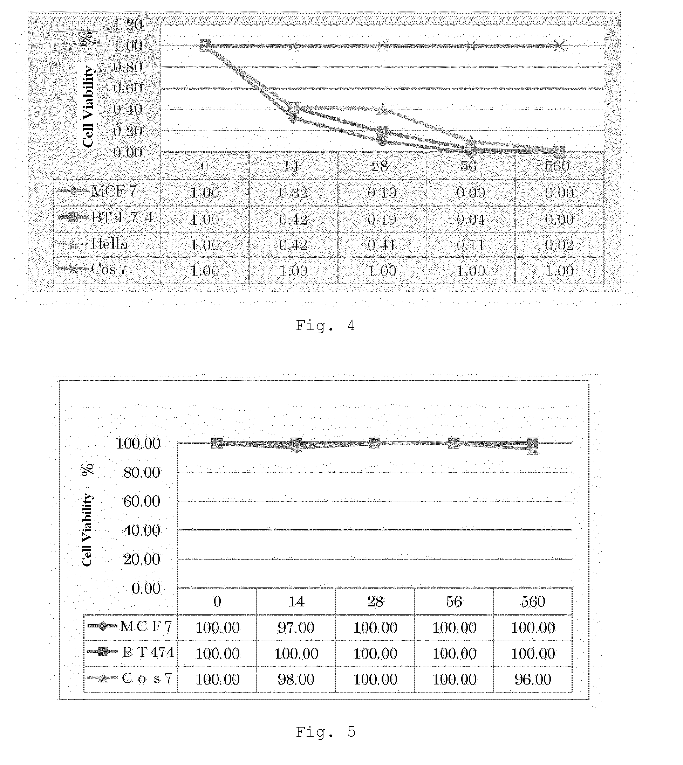

[Fig. 4] A graph showing cell viability observed 24 hours after the irradiation with a UV pulse flash (the frequency of irradiation: 0, 14, 28, 56, 560 times) (for each cell, the cell viability is 1.00 when the frequency of irradiation is 0) in Example 1
[Fig. 5] A graph showing cell viability observed 24 hours after the irradiation with a UV pulse flash from which UV-C is removed (the frequency of irradiation: 0, 14, 28, 56, 560 times) (for each cell, the cell viability is 100% when the frequency of irradiation is 0) in Example 1

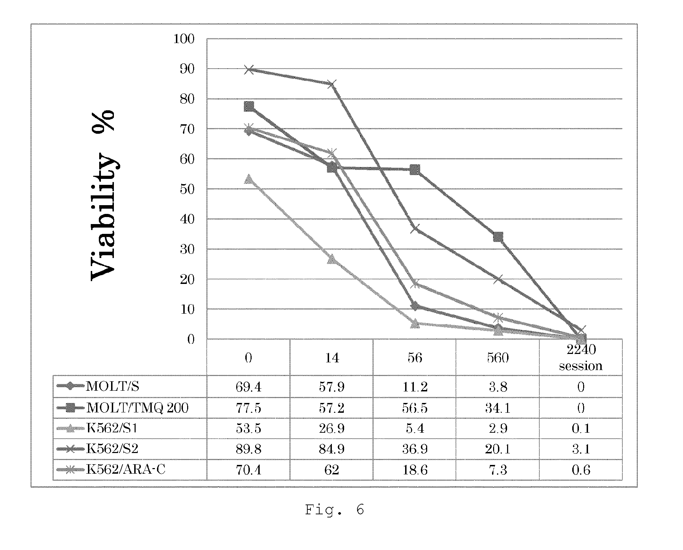

[Fig. 6] A graph showing cell viability observed 24 hours after the irradiation of human leukemia cell lines with a UV pulse flash (the frequency of irradiation: 0, 14, 56, 560, 2240 times) (for each cell, the cell viability is 100% when the frequency of irradiation is 0) in Example 2

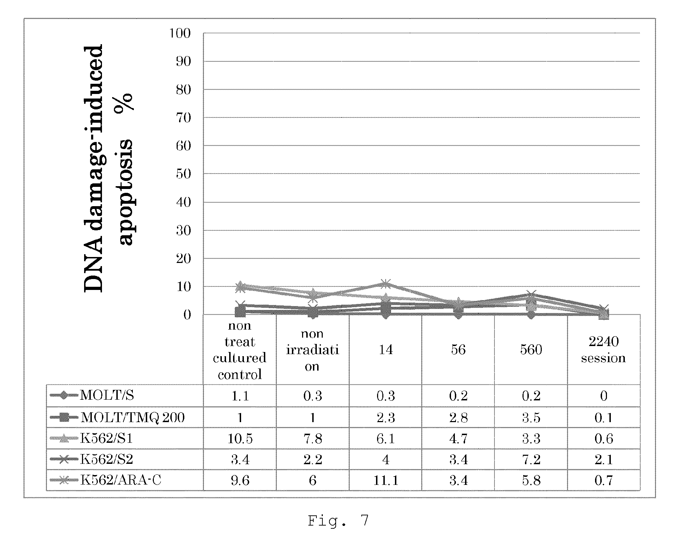

[Fig. 7] A graph showing a proportion of a cell in which early-stage apoptosis caused by DNA disorder has been induced, in Example 2

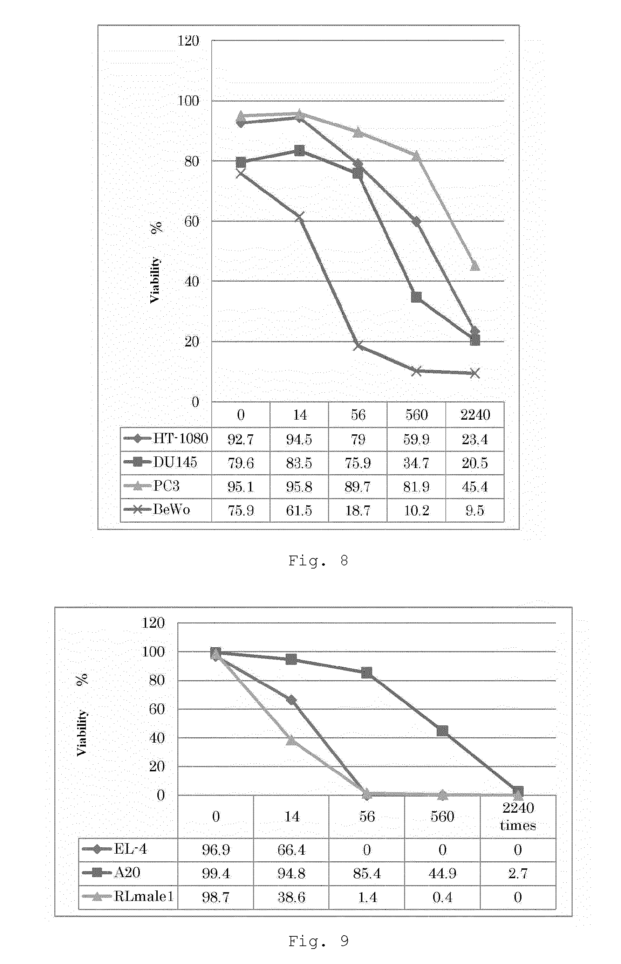

[Fig. 8] A graph showing cell viability observed 24 hours after the irradiation of human tumor cell lines (fibrosarcoma, prostate cancer and malignant chorioepithelioma) with a UV pulse flash (the frequency of irradiation: 0, 14, 56, 560, 2240 times) (for each cell, the cell viability is 100% when the frequency of irradiation is 0) in Example 3
[Fig. 9] A graph showing cell viability observed 24 hours after the irradiation of murine lymphoma cell lines with a UV pulse flash (the frequency of irradiation: 0, 14, 56, 560, 2240 times) (for each cell, the cell viability is 100% when the frequency of irradiation is 0) in Example 4

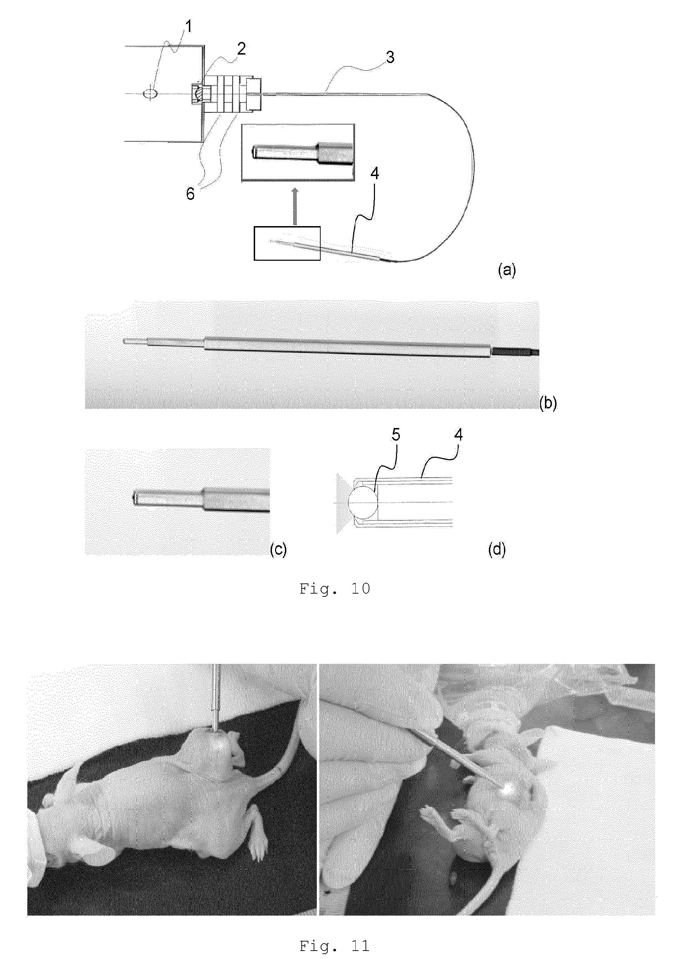

[Fig. 10] A figure showing a body surface tumor tissue irradiating apparatus 10 used in Example 5
   (a) Overall schematic view
   (b) Enlarged view of a probe 4
   (c) Enlarged view of a tip of a probe 4
   (d) Sectional view of a tip of a probe 4: A range of an irradiation angle of 75°C is shown by a color part.
[Fig. 11] A photograph showing the irradiation of a cancerous part of a cancer-bearing murine with a UV pulse flash, by contacting a tip of a probe, in Example 5

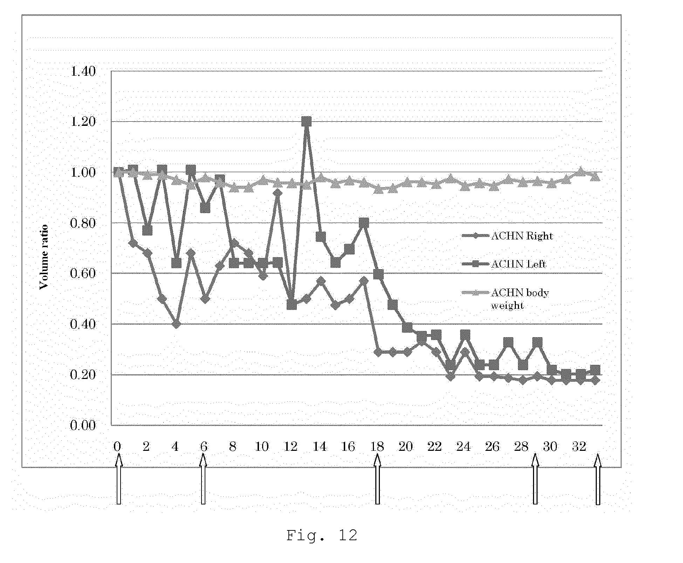

[Fig. 12] A graph showing a fluctuation of a tumor volume of an ACHN cancer-bearing murine in Example 5 (an arrow denotes a date when irradiation was performed.)

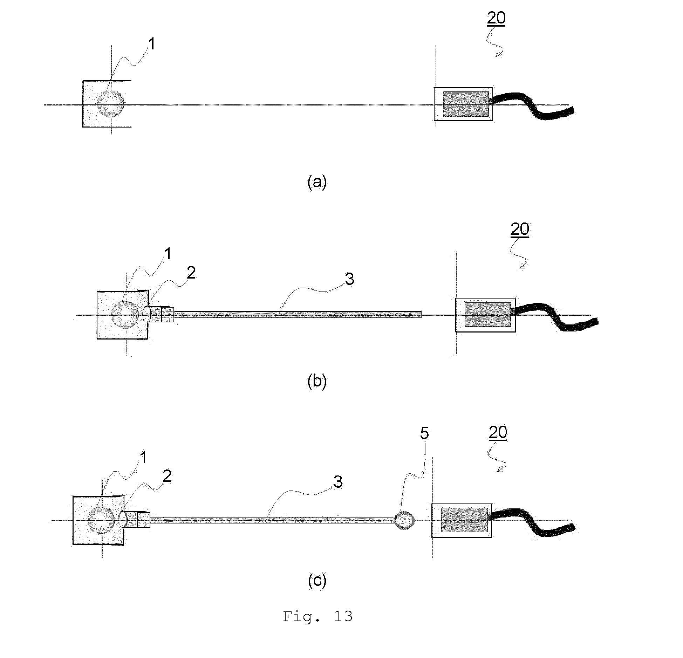

[Fig. 13] A schematic view of a method for measuring an irradiation amount in Reference Example (for details of individual components in Figure, see descriptions provided in Reference Example).

### Embodiments for Carrying Out the Invention

### -Method-

In one aspect of the present invention, there is provided a method for selectively damaging and killing tumor cells comprising irradiating tumor cells with a UV pulse flash, embodiments of which are described hereinafter.

Tumor cells to which the method of the present invention is applied are not particularly limited, and include cells of carcinoma (malignant tumor originated from epithelial tissue), sarcoma (malignant tumor originated from non-epithelial tissue), leukemia, malignant lymphoma, benign tumors, and cell lines originated from such cells. On the other hand, non-tumor cells refer to normal cells other than such tumor cells as described above. The method of the present invention is applicable to a portion in which such tumor cells and non-tumor cells are mixed.

The method of the present invention is applicable not just outside a living body (to collected or cultured cells) but also in a living body, with respect to tumor cells of a human, a non-human mammal and other animals (e.g., laboratory animals, pets). Namely, in one aspect of the present invention, there is provided a treatment method or an auxiliary surgery means for selectively damaging and killing tumor cells in a living body of a human and a non-human mammal.

The UV pulse flash used in method of the present invention has continuous emission spectra ranging at least from 230 to 270 nm, including about 265 nm, which is a DNA absorption wavelength, in an ultraviolet wavelength region corresponding to UV-C. The UV pulse flash may have continuous emission spectra covering much wider range.

An irradiation amount per unit area per one-time with the UV pulse flash [(J/cm²)/time] and the frequency of irradiation [time], that is, an accumulated irradiation amount per unit area [J/cm²], which is an integrated value thereof, are controlled to be within a range which permits the selective damaging and killing of tumor cells, according to types of tumor cells and non-tumor cells. Specifically, by controlling the accumulated irradiation amount per unit area within a specific range, substantially, it is possible to damage and kill tumor cells alone without damaging and killing non-tumor cells (even if the non-tumor cells are damaged, the damage is repairable and the non-tumor cells do not lead to cell death). An insufficient accumulated irradiation amount per unit area cannot completely kill tumor cells, while an excess of accumulated irradiation amount per unit area damages and kills or eliminates a large amount of non-tumor cells as well as tumor cells. Thus, the accumulated irradiation amount per unit area is appropriately controlled so as to accomplish an objective in terms of e.g., a viability of tumor cells or a tumor volume.

The accumulated irradiation amount per unit area [J/cm²] is a function of an output of a light source of a UV pulse flash in one-time irradiation [J/time] and the frequency of irradiation [time], that is, an integrated output [J], which is an integrated value thereof; and an area irradiated with the UV pulse flash [cm²]. Furthermore, when the light source is regarded as a point light source, the accumulated irradiation amount per unit area [J/cm²] of the UV pulse flash is inversely proportional to the square of a distance from the light source.

For example, as shown in Example 1, set forth later, in the case where a xenon flash lamp (BHX-200, COMET Corp.) is used as a light source of the UV pulse flash, and a distance between the xenon flash lamp and a portion to be irradiated is 8 cm, when the integrated output is 45 J or more, 50% or more of tumor cells (human breast cancer cultured cell lines and human cervical cancer cell lines) can be damaged and killed (44.8J (14 times), the tumor cell viability observed 24 hours after the irradiation is not more than 50%); when the integrated output is 90 J or more, most of the tumor cells can be damaged and killed (89.6J (28 times), the tumor cell viability observed 24 hours after the irradiation is about 20 to 40%); and when the integrated output is 900 J or more, the tumor cell can be almost completely eliminated. Under the above conditions, when the integrated output is not more than 20000 J, the degree of damaging and killing of non-tumor cells is low, and when the integrated output is not more than 7100 J, non-tumor cells are allowed to be viable almost completely (7168J (2240 times), the non-tumor cell viability observed 24 hours after the irradiation is nearly 100%). Thus, when the method of the present invention is applied to tumor cells such as human breast cancer cells and human cervical cancer cells, for example, the range of from 90 to 7100 J at which it is possible to damage and kill most of the tumor cells but to allow the non-tumor cell to be viable almost completely can be mentioned as a preferable range of the integrated output of the UV pulse flash under the above conditions.

In the case where the method of the present invention is applied to tumor cells other than human breast cancer cells and human cervical cancer cells, an integrated output of a higher range within the above range, or an integrated output of a range having an upper limit and/or a lower limit changed from the upper limit/lower limit of the above range may be defined as a preferable range for such tumor cells, as needed in view of its effect. For example, when the method of the present invention is applied to tumor cells which are harder to damage and kill than the human breast cancer cells and human cervical cancer cells, it is preferable that the upper limit is raised to 14200 J or the lower limit is raised to 180 J and thereby the integrated output of the UV pulse flash is controlled to be within a range of 90 to 14200 J or a range of 180 to 14200 J.

Conditions relating to the integrated output and the distance in the case where such a xenon flash lamp as described above is used in the method of the present invention are applicable even under different light source, integrated output and distance conditions, as long as the accumulated irradiation amount per unit area is a value to represent the conditions. Specifically, conditions about an integrated output, a distance and a light source may vary as long as a value corresponding to, for example, the "accumulated irradiation amount per unit area that is achieved at a distance of 8 cm from a light source having an integrated output of 90 to 7100 J" is attained.

A value of the integrated output of the xenon flash lamp (BHX-200) is a theoretical value calculated from a capacitor volume and a voltage, as described in Example 1, and represents an energy ranging across a whole region of a wavelength (for example, 200 to 1000 nm) of a light to be emitted by the xenon flash lamp. As shown in Reference Example, an energy itself originated from a wavelength of UV-C (200 to 300 nm) or a wavelength of 230 to 270 nm is part of the above integrated output, which is an energy ranging across the above whole region of the wavelength. Furthermore, the integrated output, which is an emission energy of an emission tube of the xenon flash lamp (BHX-200), can be converted to an accumulated irradiation amount per unit area of a portion that is positioned at a distance of 8 cm from the xenon flash lamp, based on the comparison as shown in Reference Example set forth later. Thus, for example, the "accumulated irradiation amount per unit area that is achieved at a distance of 8 cm from a light source having an integrated output of 90 to 7100 J" can be converted to about 70 to 5700 J/cm² in terms of an energy ranging across a whole region of a wavelength (200 to 1000 nm) (total accumulated irradiation amount per unit area), and to about 6 to 480 J/cm² in terms of an energy originated from a wavelength of UV-C (200 to 300 nm) (see Table 2). The accumulated irradiation amount per unit area that is achieved at a distance of 8 cm from light sources each having an integrated output of 180 and 14200 J can be converted to about 140 J/cm² and about 11400 J/cm², respectively, in terms of the total accumulated irradiation amount per unit area, and about 12 J/cm² and 960 J/cm², respectively, in terms of the UVC accumulated irradiation amount per unit area.

The frequency of irradiation per unit time and the treatment time using the UV pulse flash (an integrated value thereof is the above-described frequency of irradiation) are controlled to be within an appropriate range in view of the accumulated irradiation amount per unit area, conditions of the integrated output, performance of a light source, or the like. For example, by using a light source capable of emitting a UV pulse flash of not less than 1 J/time several times to some dozen times per one second, the time for the irradiation step can be controlled to be within several minutes, preferably within one minute, more preferably within 30 seconds.

The energy of the UV pulse flash may be considerably attenuated if transmitted through a glass (e.g., a slide glass, a cover glass, a condenser) or a plastic (e.g., a dish). Thus, the UV pulse flash is desirably applied directly to the tumor cells. If the condenser or the like is used, it is indispensable to use a quartz glass material through which UV-C is expected to be transmitted, and in view of the attenuation of the energy, the output should be controlled. For example, as shown in Reference Example set forth later, when an irradiation light to be emitted from a xenon flash lamp (BHX-200) is condensed at a quartz lens, goes through a quartz fiber, and is applied from a tip of a probe in which a sapphire ball is embedded, the accumulated irradiation amount per unit area at a portion to be irradiated is attenuated to some degree.

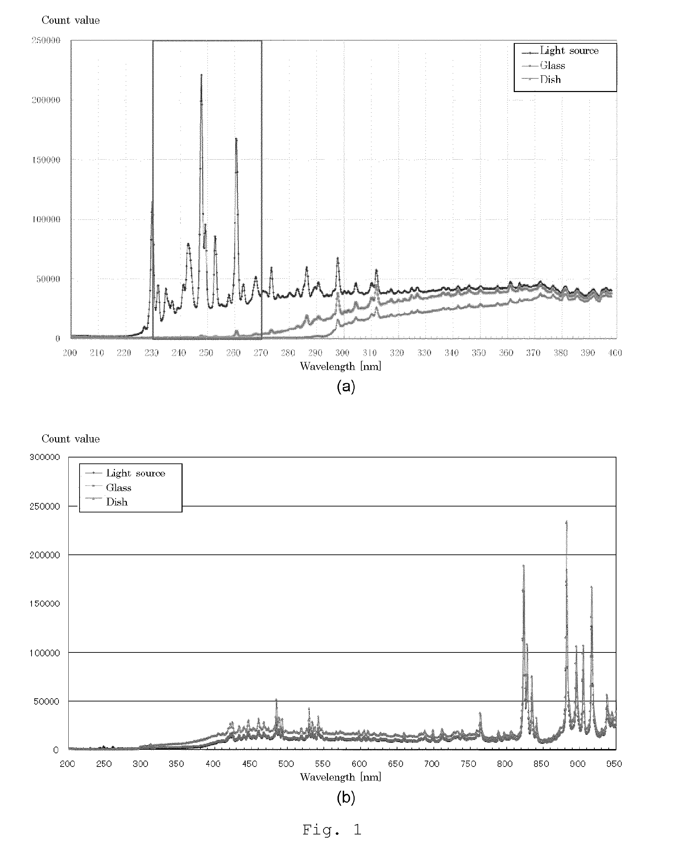

The UV pulse flash may be emitted from any light source as long as being able to satisfy the above conditions, but preferred is, for example, a pulse light to be emitted from a xenon flash lamp. The xenon flash lamp can emit a flash from a xenon gas that has high continuous spectra ranging from an ultraviolet region to an infrared region (see Fig. 1) with an instantaneous high peak output for several times to some dozen times per one second. For the method of the present invention, it is also possible to employ irradiating apparatus for light pulse sterilization comprising a xenon flash lamp (for example, BHX-200, COMET Corp.) or modified products thereof, which are employed in the field of food or the like.

Conventional low-pressure mercury lamps have emission spectra having an intensive peak only at a specific wavelength, e.g., 254 nm, and do not have continuous emission spectra ranging from 230 to 270 nm, and therefore do not provide sufficient UV energy. The irradiation with the UV light so as to fulfill accumulated irradiation amount per unit area using the low-pressure mercury lamp takes a long hours, resulting in giving considerable damage not just to tumor cells but also to non-tumor cells and thus failing to selectively damage and kill the tumor cells.

### -Apparatus-

In one aspect of the present invention, there is provided an apparatus for medical treatment, more specifically an apparatus for treating tumor tissues, which is capable of performing the method of the present invention.

The apparatus of the present invention comprises a light source of a pulse light having continuous emission spectra ranging at least from 230 to 270 nm (UV pulse flash). As the light source, a xenon flash lamp is preferred, as is described with regard to the method of the present invention.

As the apparatus of the present invention, there can be mentioned, for example, an endoscope, a laser microscope, and a body surface tumor tissue irradiating apparatus. These apparatuses comprise a structure for applying a UV pulse flash from a light source (preferably, a configuration counter capable of controlling an output of the light source is mounted to a component such as a power source). With regard to other structures, structures of conventional endoscopes, laser microscopes, body surface tumor tissue irradiating apparatus are applicable optionally with appropriate modification.

In the case of the endoscope, for example, in addition to a light for illumination, the UV pulse flash to be emitted from a light source provided at a control device side outside a living body, is led through an optical fiber, and is applied from a tip to a part to be treated. In this case, in order to lead the UV light into the living body without attenuation, a material of the fiber is preferably quartz glass or a material having a specular structure therein. Furthermore, it is desired to develop an apparatus in which the tip of the endoscope has a small-sized UV pulse light oscillating component therein.

In the case of the laser microscope, separately from a laser serving as a light source, the UV pulse flash is applied to a part to be observed and to be treated at a level of individual cells. A lens optical system used at this time being shared by observation use inevitably requires the use of a quartz glass material as a lens, which is expected to cost quite high, and thus it is preferable to provide an optical path exclusive for the UV separately from the optical path for observation, and to switch between these optical paths.

In the case of the body surface tumor tissue irradiating apparatus, the UV pulse flash is applied to a part to be treated after led from a movable unit equipped with a light source or a quartz fiber irradiating apparatus (the light is condensed at a quartz lens and goes through a quartz fiber, and is applied from a probe having quartz or a sapphire ball with its tip having an integrating sphere performance). In another embodiment, at the time of craniotomy/celitomy surgery, after surgical tumor extracting surgery, the UV pulse light can be applied to the extracted part to eliminate remaining tumors. Furthermore, by making the tip of the probe serve as a blade (as is the case with a tip of an injection needle), the apparatus can be inserted into a living body.

### Examples

### [Example 1]

### Material/Method

UV pulse flash light source (using a xenon flash lamp) : BHX-200 (COMET Corp.)
Confocal laser scan microscope: LSM510-META (Carl Zeiss MicroImaging, Jena Germany)

### Tumor cells:

1. MCF-7 (cell line originated from human breast cancer)
2. BT474 (cell line originated from human breast cancer)
3. Hella (cell line originated from human cervical cancer) Non-tumor cells:

1. Cos7 (cell line originated from African green monkey kidney)
2. MDCK (cell line originated from canine kidney uriniferous tubule epithelial cell)
At first, utilizing cell intrinsic fluorescence of each cell, morphology information of each cell was obtained. Then, to each cell, MBL Azami-Green (phmAG1-MC1) DNA was transfected using Lipfectamine2000 (Invitrogen), and the fluorescence was observed. Under the environment of 5% CO₂ and 37°C, three-dimensional images were obtained using LSM510-META. Thereafter, BHX-200 was set at a position of 8 cm immediately above the cells, and the cells were irradiated with a UV pulse flash (the frequency of irradiation: 1, 5, 10, 14, 28, 56, 560, 1120, 2240, 3360, 6720 times). Thereafter, three-dimensional images were obtained again. One-time emission energy (output) was 3.2 J (1/2 x capacitor volume (C) x voltage (V)² =1/2 x (7.5 x 10⁻⁶) x 920² ≈ 3.2), and the frequency of emission per one second was 56 times. At the same time, to the cells, propidium iodide was added to observe the life and death of the cells. After the irradiation with the UV pulse flash, the cells were cultured continuously for 24 hours. Then, the cells were observed again to determine a viability.

### Result

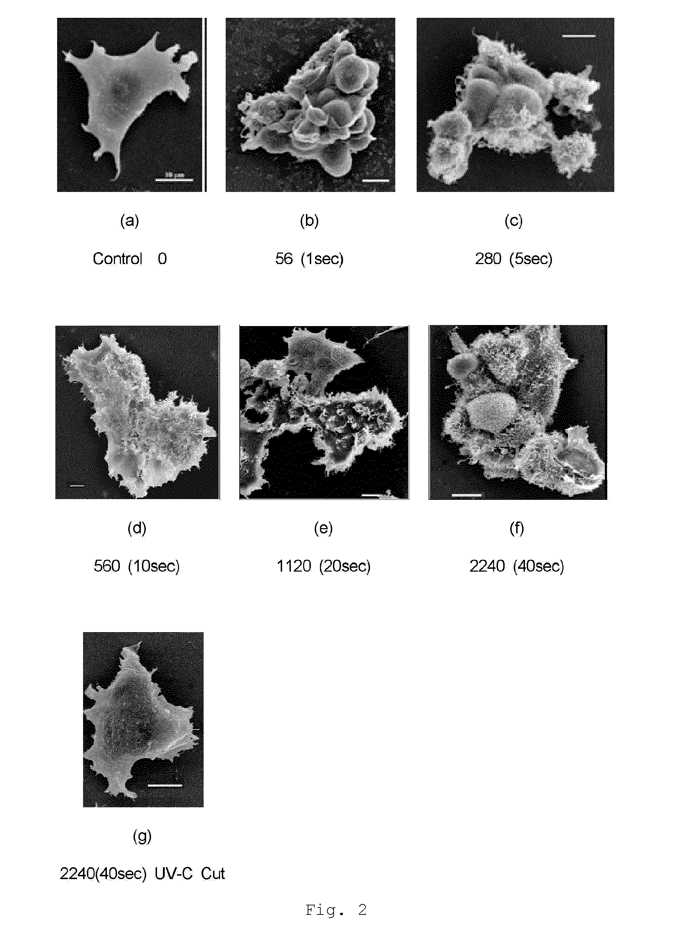

As a result of intrinsic fluorescence observation based on finger printing method using LSM510-META, and Azami-Green fluorescence observation (see Fig. 3), in all of the cells employed in the experiment, cell atrophy was started to be recognized from the irradiation at a frequency of one time. At the irradiation at a frequency of 56 times, morphology believed to result from the change of the membrane structure of the cell surface was observed, and cell atrophy was conspicuous. In scanning electronic microscope observation (see Fig. 2) as well, cell atrophy and the change of cell membrane structure were similarly recognized.

With regard to the cell viability observed 24 hours after the irradiation, the viability of the non-tumor cells continued to be 100% until the irradiation at a frequency of 560 times, while the viability of the tumor cells was dropped to about 40% by the irradiation at a frequency of 14 times, dropped to 20% except for Hella cell (40%) by the irradiation at a frequency of 28 times, and reached almost 0% by the irradiation at a frequency of 560 times (see Fig. 4). That is, the irradiation at a distance of 8 cm from the light source with a UV pulse flash in an amount of 1.792 kJ (= (3.2 J)/time x 560 times) allowed the non-tumor cells to be viable at a percentage of 100%, but eliminated the tumor cells. Judging from the form of the nucleus, apoptosis was also induced. In the case of the irradiation for 120 seconds (6720 times), the non-tumor cells were observed to have changed membrane structure and partial cell death as well.

On the other hand, in an experiment group in which a plastic cover (thickness: 1.25 m) of a dish and a CO₂ incubator plastic cover (thickness: 1.2 mm) of a laser microscope were inserted into an observation optical path to remove UV-C (not more than 290 nm) (see Fig. 1), no marked difference in viability was recognized between the tumor cells and non-tumor cells (see Fig. 4).

### Discussion

The result of the irradiation with the pulse light from which UV-C was removed strongly suggested that a visible light and a near infrared light were not involved but UV-C was involved with the present phenomenon. It is presumed that the tumor cells, by being irradiated with the UV pulse flash, underwent, for a short period of time, evident cell morphology change (atrophy) and cell membrane disruption, discharging substances within cytoplasm to the outside of the cells, resulting in cell death, while tumor cells which escaped immediate death also resulted in cell death due to apoptosis induced by the formation of DNA pyrimidine dimer.

Furthermore, when the cell death is studied based on morphology observation, it is found that the tumor cells, because of having an ultraviolet sensitivity evidently higher at a UV-C region as compared with the non-tumor cells, underwent cytoclasis. Namely, at a low ultraviolet exposure dose, the tumor cells alone underwent cell death, but the non-tumor cells did not lead to cell death. This is presumed to be attributable to the presence of ultraviolet receptors (absorbers) specific to the tumor cells, and the possibility of their increasing the ultraviolet sensitivity is suggested. It is presumed that the receptors are different from one another depending on tumor cells, and are related to a compositional change in glycolipid and glycoprotein glycans with the canceration and the malignant transformation of the cells.

### [Example 2]

### Tumor cell lines

4. Human leukemia cell line MOLT/S
5. Human leukemia cell line MOLT/TMQ 200 (line 200-time resistant to trimetrexate (TMQ) anticarcinogenic agents)
6. Human leukemia cell line K562/S1
7. Human leukemia cell line K562/S2
8. Human leukemia cell line K562/ARA-C (line resistant to Cytarabine anticarcinogenic agent)

### Method

To each cell described above, propidium iodide (PI) was added. At first, utilizing intrinsic fluorescence of each cell, morphology information of each cell was obtained, similarly to Example 1. Then, under the environment of 5% CO₂ and 37°C, three-dimensional images were obtained using LSM510-META. Thereafter, BHX-200 was set at a position of 8 cm immediately above the cells, and the cells were irradiated with a UV pulse flash (the frequency of irradiation: 0, 14, 56, 560, 2240 times). Thereafter, three-dimensional images were obtained again. The life and death of the cells was observed based on PI reaction. After the irradiation with the UV pulse flash, the cells were cultured continuously for 24 hours. The cells were observed again to determine a viability. Then, based on the analysis by flow cytometry (FACS Aria, Japan Becton, Dickinson and Company, the number of cells=5 x 10⁵, n=3), a cell viability was determined. Results are set forth in Fig. 6.

As such, the present invention was found to be applicable not just to solid cancers but also to hematological cancers. At the time of clinical application, the application is believed to cover an extremely wide range. For example, on the principle of renal dialysis, human blood is led to an external UV pulse flash apparatus, where the blood is subjected to the UV irradiation, and then the blood is returned to the body, whereby the hematological cancer is treatable by the apparatus. In addition, while both MOLT/TMQ 200 and K562/ARA-C are resistant to anticarcinogenic agents and are cancers which are not expected to be treatable by the effect of the anticarcinogenic agents, since the working mechanism of the present invention is based not on pharmacological damaging but on physical damaging, the present invention is found to have enormous effect also on these cancers as well as other cancers. That is, the method of the present invention having the novel working mechanism, which is expected to have an effect also on cancers difficult to treat, is expected to be able to become a good seed for patients.

Moreover, for each cell, an effect of the irradiation with the UV pulse flash at a prescribed frequency in the same manner as described above on early-stage apoptosis (DNA disorder) was studied. Using Annexin V as a marker, the early-stage apoptosis was analyzed by flow cytometry, as was performed in connection with the above-described life and death of cells. At an early stage of apoptosis, phosphatidylserine (PS) lying inside of the cell membrane expresses to the outside of the cell membrane, and is bonded with Annexin V, which has a high affinity for PS. Annexin V is a protein of 35-36kDa which bonds with phospholipid based depending on Ca⁺⁺. The expression of PS to the outside of the cell membrane is followed by the loss of a normal cell membrane structure and the initiation of the fragmentation of DNA or the agglomeration of chromatin. Based on this principle, by detecting a bond with Annexin V, the early-stage apoptosis cell is recognized. Meanwhile, a subject was separately prepared as a no-treatment control (non treat cultured control), and was subjected to flow cytometry analysis 24 hours before the experiment. Results are set forth in Fig. 7. A ratio of inducing apoptosis was almost not more than 10%, and thus it is considered that the involvement of apoptosis in the working mechanism in the present invention is low.

### [Example 3]

### Tumor cell lines

9. Human fibrosarcoma cell line HT-1080
10. Human prostate cancer cell line DU145
11. Human prostate cancer cell line PC3
12. Human malignant chorioepithelioma cell line BeWo Method
To each cell described above, propidium iodide (PI) was added. At first, utilizing intrinsic fluorescence of each cell, morphology information of each cell was obtained, similarly to Example 1. Then, under the environment of 5% CO₂ and 37°C, three-dimensional images were obtained using LSM510-META. Thereafter, BHX-200 was set at a position of 8 cm immediately above the cells, and the cells were irradiated with a UV pulse flash (the frequency of irradiation: 0, 14, 56, 560, 2240 times). Thereafter, three-dimensional images were obtained again. The life and death of the cells was observed based on PI reaction. After the irradiation with the UV pulse flash, the cells were cultured continuously for 24 hours. The cells were observed again to determine a viability. Then, based on the analysis by flow cytometry (FACS Aria, Japan Becton, Dickinson and Company, the number of cells=5 x 10⁵, n=3), a cell viability was determined. Results are set forth in Fig. 8.

These are sarcoma/cancer classified as being, so-called, malignant, and are considered to be able to be eliminated by a larger exposure dose of UVC (965 J/cm²) than the dose in the case of general cancer cells. This amount is lower than the UVC exposure amount that can cause disorder in normal cells (1447 J/cm²)_{.}

### [Example 4]

### Tumor cell lines

13. Murine lymphoma EL-4
14. Murine lymphoma A20
15. Murine lymphoma RL male 1

### Method

To each cell described above, propidium iodide (PI) was added. At first, utilizing intrinsic fluorescence of each cell, morphology information of each cell was obtained, similarly to Example 1. Then, under the environment of 5% CO₂ and 37°C, three-dimensional images were obtained using LSM510-META. Thereafter, BHX-200 was set at a position of 8 cm immediately above the cells, and the cells were irradiated with a UV pulse flash (the frequency of irradiation: 0, 14, 56, 560, 2240 times). Thereafter, three-dimensional images were obtained again. The life and death of the cells was observed based on PI reaction. After the irradiation with UV pulse flash, the cells were cultured continuously for 24 hours. The cells were observed again to determine a viability. Then, based on the analysis by flow cytometry (FACS Aria, Japan Becton, Dickinson and Company, the number of cells=5 x 10⁵, n=3), a cell viability was determined. Results are set forth in Fig. 9.

As was the case with human leukemia cells, with regard to the murine lymphoma cells, similar results were obtained. This suggested the applicability to humoral cancer not just in a human but also in other animals.

### [Example 5]

### Body surface tumor tissue irradiating apparatus

A body surface tumor tissue irradiating apparatus 10, as shown in Fig. 10, was prepared. A UV pulse flash light from a UV pulse light source 1 (QSO-7016UVSP, a UVC oscillating tube used in BHX-200) can be applied in such a manner that the UV pulse flash light is lighted and condensed at a quartz lens 2 (focal distance at the front side: 26 mm, focal distance at the back side: 25 mm), a focal place thereof having a lighting opening of a quartz fiber 3 of 1.0 mm in diameter set thereon, and the UV pulse flash light is led, through the quartz fiber 3 of 1200 mm in length, to a tip of a probe 4. The tip of the probe 4 had a sapphire ball 5 of 1. 5 mm embedded therein to ensure an irradiation angle of 75°C.

### Cancer-bearing murine

BALB/cA-nu/nu female: at six weeks past the birth, ACHN (human kidney cancer cell line) was subcutaneously implanted to initiate a cancer-bearing state.

### Method

After the tumor of the above cancer-bearing murine became swollen with its diameter being changed from 20 to 30 mm, an irradiation experiment using the above body surface tumor tissue irradiating apparatus was initiated. On 0, 6th, 18th, 29th and 33th day, a cancerous part was contacted with a tip of a probe and thereby irradiated with a UV pulse flash (see Fig. 11). At the same time, as a control, a non-tumor skin was irradiated in the same exposure amount. A fluctuation of the tumor volume of the cancer-bearing murine is shown in Fig. 12. A numeral pointed by an arrow denotes a day when the irradiation was performed. The tumor size of the cancer-bearing was 942 rm³ and 1308.33 mm³, which were quite large tumor bulks as shown in Fig. 11, and the body weight was 25.8 g. The volume started to decrease from the first day, and recorded the first lowest value on the fourth day. On the following day, the volume showed increase tendency, and thus a second irradiation was performed on the sixth days. While seeing the fluctuation of the volume, the irradiation was performed until a fifth irradiation, and by way of surgery, a tumor bulk (remains) was extracted. At the same time, a formalin paraffin specimen and an electronic microscope specimen were prepared for morphological consideration. As a result, it was found that the tumor cells underwent necrosis, melting and disappearance, while fibrous tissues were present so as to surround the tumor cells. The reason why the volume ratio became flat at about 80% decrease is believed to be that the disappearance of the tumor cells was replaced by the appearance of fibrous tissues during repairing process. The fibrous tissues are normal cells, and thus are not influenced even if irradiated with the UV pulse flash. In addition, it can be interpreted from almost no change in the body weight and the maintenance of the initial body weight that the elimination of the tumor cells allowed nourishment that was supposed to be exploited by the tumors to be used for the maintenance of the living body, resulting in the maintenance of the body weight. In a group of the irradiation of the normal skin prepared as a control, no morphology change was observed.

### [Reference Example]

### Measurement conditions for irradiation amount

[1] Pulse light source: QSO-70106UVSP (BHX-200)
   Stroboscope power source: a device corresponding to HD-200
[2] Condenser
[3] Quartz fiber of 1200 mm in length and 1 mm in diameter
[4] Sapphire ball
[20] Measurement device
   Light receiving part: UV module H8496-11 of 8 mm in diameter, manufactured by Hamamatsu Photonics K.K.
   Measurement part: DEF-2100, manufactured by Kyoritsu Electric Co., Ltd.
   Optical bench: X type, 1000 mm in length

(1) An emission energy of an emission tube of a UV pulse flash light source "QSO-70106UVSP" (BHX-200, COMET Corp., 56Hz/s emission) was calculated. Result is set forth in Table 1. In all of the following tables, 1 time means 1 pulse. "All energy" per one time is a value obtainable from the equation: 1/2 x capacitor volume (C) x voltage (V)². "UVC energy" per one time is a value obtainable based on the "All energy" from the analysis of the component of the wavelength.

[Table 1]

**Table 1: Emission energy of an emission tube**

| Unit: J | | | | | | | |
|---|---|---|---|---|---|---|---|
| | 1 time | 14 times | 56 times | 560 times | 2240 times | 4480 times | 6720 times |
| All energy (200 to 1000 nm) | 3.20 | 44,80 | 179.20 | 1792.00 | 7168.00 | 14336.00 | 21504.00 |
| UVC. energy nm) (200 to 300 nm) | 0.27 | 3.78 | 15.12 | 151.20 | 604.80 | 1209.60 | 1814.40 |

(2) As shown in Fig. 13(a), an irradiation amount at a distance of 80 mm from the emission tube (corresponding to the exposure dose of the cells in Examples 1 to 4) was measured. Result is set forth in Table 2.

[Table 2]

**Table 2: Irradiation amount at a distance of 80 mm from an emission tube**

| Unit: J/cm² | | | | | | | |
|---|---|---|---|---|---|---|---|
| | 1 time | 14 times | 56 times | 560 times | 2240 times | 4480 times | 6720 times |
| All energy (200 to 1000 nm) | 2.55 | 35.74 | 142.96 | 1429.65 | 5718.59 | 11437.18 | 17155.76 |
| UVC energy (200 to 300 nm) | 0,22 | 3.02 | 12.06 | 120.63 | 482.50 | 965.01 | 1447.51 |

(3) As shown in Fig. 13(b), an irradiation energy of an irradiation opening of the quartz fiber of 1200 mm in length and 1 mm in diameter was measured. A distance between the irradiation opening and a sensor was 4 mm. Result is set forth in Table 3.

[Table 3]

**Table 3: Irradiation energy of an irradiation opening of a quartz fiber of 1200 mm in length and 1 mm in diameter**

| Unit: J/cm² | | | | | | | |
|---|---|---|---|---|---|---|---|
| | 1 time | 14 times | 56 times | 560 times | 2240 times | 4480 times | 6720 times |
| All energy (200 to 1000 nm) | 2.58 | 36.12 | 144.48 | 1444.8 | 5779.2 | 11558.4 | 17337.68 |
| UVC energy (200 to 300 nm) | 0.218 | 3.052 | 12.208 | 122.08 | 488.32 | 976.64 | 1464.96 |

(4) As shown in Fig. 13(c), an irradiation energy of the quartz fiber of 1200 mm in length and 1 mm in diameter + a sapphire ball probe (corresponding to the exposure dose of the tumors in Example 5) was measured. A distance between the irradiation opening and a sensor was 4 mm. Result is set forth in Table 4.

[Table 4]

**Table 4: Irradiation energy of a quartz fiber of 1200 mm in length and 1 mm in diameter + a sapphire ball probe**

| Unit: J/cm² | | | | | | | |
|---|---|---|---|---|---|---|---|
| | 1 time | 14 times | 56 times | 560 times | 2240 times | 4480 times | 6720 times |
| All energy (200 to 1000 nm) | 1.80 | 25.30 | 101.21 | 1012.14 | 4048.58 | 8108.80 | 12145.73 |
| UVC energy (200 to 300 nm) | 0.15 | 2.14 | 8.546 | 85.456 | 341.82 | 683.64 | 1025.47 |

### Description of Marks

1: UV pulse light source
2: quartz lens
3: quartz fiber
4: probe
5: sapphire ball
6: filter guide
10: body surface tumor tissue irradiating apparatus
20: irradiation amount measurement device

## Claims

1. A method for selectively damaging and killing tumor cells comprising a step of irradiating tumor cells with a pulse light (hereinafter, also referred to as a "UV pulse flash") having continuous emission spectra ranging at least from 230 to 270 nm, outside a living body of a human or a living body of a non-human animal or in a living body of a non-human animal.

2. The method according to claim 1, wherein the UV pulse flash has an accumulated irradiation amount per unit area that is achieved at a distance of 8 cm from a light source having an integrated output of 90 to 7100 J.

3. The method according to claim 1, wherein the UV pulse flash has an accumulated irradiation amount per unit area that is achieved at a distance of 8 cm from a light source having an integrated output of 90 to 14200 J.

4. The method according to claim 1, wherein the UV pulse flash has an accumulated irradiation amount per unit area that is achieved at a distance of 8 cm from a light source having an integrated output of 180 to 14200 J.

5. The method according to claim 1, wherein the UV pulse flash has an accumulated irradiation amount per unit area of 6 to 480 J/cm² in terms of an energy originated from a wavelength of UVC.

6. The method according to claim 1, wherein the UV pulse flash has an accumulated irradiation amount per unit area of 6 to 960 J/cm² in terms of an energy originated from a wavelength of UVC.

7. The method according to claim 1, wherein the UV pulse flash has an accumulated irradiation amount per unit area of 12 to 960 J/cm² in terms of an energy originated from a wavelength of UVC.

8. The method according to any one of claims 1 to 7, wherein the step of irradiating with the UV pulse flash is carried out within 1 minute.

9. The method according to any one of claims 1 to 8, wherein the UV pulse flash is emitted from a xenon flash lamp.

10. An apparatus for treating tumor tissues comprising a light source of a pulse light having continuous emission spectra ranging at least from 230 to 270 nm (UV pulse flash).

11. The apparatus according to claim 10, which is an endoscope, a laser microscope, or a body surface tumor tissue irradiating apparatus.

12. The apparatus according to claim 10 or 11, wherein the light source is a xenon flash lamp.
